# DEMANDE DE BREVET EUROPEEN

(11) **EP 1 247 516 A2**
(43) Date de publication de la demande: **09.10.2002**
(21) Numéro de dépôt: 02290767.9
(22) Date de dépôt: 27.03.2002
(51) Int. Cl.: A61K 7/043

(54) **Composition cosmétique thermoréticulable**

(30) Priorité: 06.04.2001 FR 0104681
(71) Demandeur: L'OREAL, 75008 Paris (FR)
(72) Inventeur: Bernard, Pascale, 94370 Sucy-en-Brie (FR); Ramin, Roland, 75014 Paris (FR); Mondet, Jean, 93600 Aulnay-sous-Bois (FR)
(74) Mandataire: Casalonga, Axel

(57) **Abrégé**

L'invention concerne une composition cosmétique thermoréticulable comprenant, dans un milieu cosmétiquement acceptable, (a) au moins un composé comportant au moins deux fonctions à hydrogène labile, et (b) au moins un composé comportant au moins deux fonctions isocyanate bloquées, susceptibles d'être débloquées par chauffage, la fonctionnalité moyenne du système, c'est-à-dire le nombre total de fonctions à hydrogène labile et de fonctions isocyanate bloquées rapporté au nombre total de molécules de composés (a) et (b), étant strictement supérieure à 2, ainsi qu'un procédé de revêtement de substrats kératiniques utilisant une telle composition.

## Description

La présente invention concerne des compositions cosmétiques thermoréticulables, en particulier des vernis à ongles thermoréticulables, ainsi qu'un procédé de revêtement des matières kératiniques utilisant ces compositions.

Dans le domaine des vernis à ongles, le film déposé sur les ongles est généralement obtenu par simple séchage - n'impliquant aucune réaction chimique - de compositions cosmétiques contenant principalement des polymères filmogènes et des solvants organiques volatiles, et éventuellement des pigments ou colorants.

Ces dépôts ne présentent pas toujours une tenue satisfaisante et présentent l'inconvénient de nécessiter un renouvellement à des intervalles rapprochés ce qui représente une contrainte désagréable pour l'utilisatrice.

La demanderesse s'est fixé pour objectif de mettre au point des compositions cosmétiques, et en particulier des vernis à ongles, susceptibles d'être réticulées *in situ* et de former ainsi des films présentant une meilleure résistance chimique et mécanique que ceux de l'art antérieur.
Dans cette perspective, la demanderesse n'envisageait pas l'utilisation de deux réactifs conservés dans des récipients séparés et mélangés immédiatement avant l'application, car un tel dispositif est très peu pratique.

Le stockage, dans un même récipient, de deux réactifs susceptibles de réagir l'un avec l'autre à un moment précis posait alors le problème de la réticulation prématurée du système qu'il s'agissait à tout prix d'éviter.

La demanderesse a résolu ce problème en inactivant de manière réversible, par un agent de blocage, un des deux types de fonctions réactionnelles impliquées dans la réaction de réticulation. Cet agent de blocage est éliminé, après application de la composition cosmétique sur le substrat, par l'action de la chaleur et les fonctions réactives ainsi libérées peuvent alors réagir avec les fonctions "coréactives" également présentes dans la composition.

La présente invention a par conséquent pour objet une composition cosmétique thermoréticulable comprenant, dans un milieu cosmétiquement acceptable, au moins un premier composé (a) comportant au moins deux fonctions à hydrogène labile, et au moins un deuxième composé (b) comportant au moins deux fonctions isocyanate bloquées, susceptibles d'être débloquées par chauffage, la fonctionnalité moyenne du système, c'est-à-dire le nombre total de fonctions à hydrogène labile et de fonctions isocyanate bloquées rapporté au nombre total de molécules de composés (a) et (b), étant strictement supérieure à 2.

L'invention a également pour objet un procédé de revêtement d'un substrat kératinique, comprenant le chauffage du film cosmétique déposé à une température suffisante pour provoquer le déblocage d'une partie ou de la totalité des fonctions isocyanate bloquées portées par le composé (b), de manière à permettre la réticulation du dépôt.

Ces compositions cosmétiques thermoréticulables combinent une bonne stabilité dans le temps à température ambiante et une excellente réactivité après chauffage permettant un durcissement rapide des films déposés.

Comme indiqué ci-dessus, les compositions cosmétiques thermoréticulables contiennent deux types de composés :
un premier composé, appelé composé (a), comportant au moins deux fonctions à hydrogène labile, et
un deuxième composé, appelé composé (b), comportant au moins deux fonctions isocyanate inactivées par la présence un agent de blocage et susceptibles d'être débloquées, c'est-à-dire activées, sous l'effet de la chaleur.

Pour que ce système réactif formé par les composés (a) et (b) puisse former un réseau macromoléculaire réticulé, sa fonctionnalité moyenne, c'est-à-dire le nombre total de fonctions à hydrogène labile et de fonctions isocyanate bloquées rapporté au nombre total de molécules de composés (a) et (b), doit être supérieure à 2. En effet, une fonctionnalité moyenne inférieure ou égale à 2 donnerait simplement un système polymérique linéaire ou ramifié.

Pour l'obtention d'un effet de réticulation satisfaisant, la fonctionnalité moyenne du système réticulant des compositions cosmétiques de la présente invention est de préférence au moins égal à 2,2 et mieux encore comprise entre 2,5 et 100.

Dans un mode de réalisation des compositions cosmétiques de la présente invention, une fraction plus ou moins importante ou la totalité des composés (a) et/ou (b) peuvent porter respectivement, en plus des fonctions à hydrogène labile et/ou des fonctions isocyanate bloquées qui leur sont propres, une ou plusieurs fonctions "coréactives". Autrement dit, une partie ou la totalité des composés (a) peuvent porter, en plus des fonctions à hydrogène labile, un certain nombre de fonctions isocyanate inactivées par un agent de blocage et, de manière analogue, une partie ou la totalité des composés (b) peuvent porter, en plus des fonctions isocyanate bloquées, un certain nombre de fonctions à hydrogène labile.
Cela signifie que la présente invention englobe, dans un mode de réalisation particulier, également les compositions dans lesquelles l'ensemble des composés formant le système de réticulation comportent à la fois des fonctions à hydrogène labile et des fonctions isocyanate bloquées.

Selon la présente invention, les fonctions à hydrogène labile sont choisies de préférence parmi les fonctions amine primaire (-NH₂), amine secondaire (>NH), hydroxyle (-OH), acide carboxylique (-COOH) ou thiol (-SH).
Parmi ces fonctions, on préfère tout particulièrement les fonctions amine primaire et secondaire et les fonctions hydroxyle.

Les fonctions isocyanate bloquées susceptibles de réagir, après activation thermique, avec les fonctions à hydrogène labile, répondent de préférence à la formule

-NH-C(=O)-B

dans laquelle B représente un radical dérivé d'un agent de blocage BH choisi parmi les composés organiques comportant un ou plusieurs, et de préférence un seul atome d'hydrogène labile.
Les agents de blocage doivent être capables d'empêcher la réaction ultérieure des groupes isocyanate à température ambiante, ou plus généralement à une température inférieure à 45 °C, avec toute autre molécule contenant des atomes d'hydrogène labiles, mais doivent permettre cette réaction à une température plus élevée, c'est-à-dire généralement supérieure ou égale à 50 °C, après déblocage thermique de la fonction isocyanate.

On peut citer à titre d'exemples d'agents de blocage appropriés
a) les alcools, notamment les monoalcools, comportant de 1 à 10 atomes de carbone, de préférence des monoalcools tertiaires comportant de 4 à 10 atomes de carbone et en particulier de 4 à 6 atomes de carbone,
b) les phénols, notamment les monophénols, comportant notamment de 6 à 50 atomes de carbone, de préférence de 6 à 20 atomes de carbone, tels que l'ortho-crésol, le para-crésol, le 2,6-diméthylphénol, le 2-tert-butylphénol, le 2-[(diméthylamino)méthyl]phénol et le salicylate de méthyle,
c) les amides et en particulier les amides cycliques, comportant notamment de 3 à 50 atomes de carbone, telles que le caprolactame, le méthylacétamide, les imides tels que le succinimide et les succinimides substitués notamment par un ou deux radicaux alkyle en C₁₋₆,
d) les oximes, notamment ceux en C₂₋₅, tels que l'acétone-oxime, la méthylisopropylcétone-oxime, la méthylisobutylcétone-oxime, la diisobutylcétone-oxime et la diisopropylcétone-oxime,
e) les composés β-dicarbonyle, notamment les β-diesters, les β-dicétones et les β-esterscétones, tels que les malonates de dialkyle en C₁₋₆, par exemple le malonate de diéthyle, les acétoacétates d'alkyle en C₁₋₆, par exemple l'acétoacétate d'éthyle ou l'acétoacétate de tert-butyle, ou encore la 2,4-pentanedione,
f) les pyrazoles, en particulier le 3-méthylpyrazole et le 3,5-diméthylpyrazole,
g) les esters d'acide hydroxyamique et d'alcools en C₁₋₆,
h) les triazoles tels que le benzotriazole,
i ) les imidazolines telles que la 2-phénylimidazoline, la 2,4-diméthylimidazoline et la 4-méthylimidazoline,
j) les tétrahydropyrimidines, et
k) les imidazoles tels que le 2-éthyl-4-méthylimidazole.

Ces réactifs de blocage et les conditions réactionnelles appropriées sont décrits par exemple dans les articles : "Blocked Isocyanates" de Zeno W. dans *Progress in Organic Coatings,* 3, pages 73 - 99 (1975) et "New Developments in the Field of Blocked Isocyanates" Zeno W., dans *Progress in Organic Coatings*, 9, pages 3 - 28 (1981).

Les réactions d'inactivation et d'activation des fonctions isocyanate portées par le ou les composés (a), c'est-à-dire la réaction entre les fonctions isocyanate libres et l'agent de blocage BH forment un équilibre réactionnel. Pour empêcher que les fonctions isocyanates réactives, libérées par chauffage, ne réagissent de nouveau avec l'hydrogène labile de l'agent de blocage, c'est-à-dire pour déplacer l'équilibre réactionnel vers l'activation des fonctions isocyanate, il est souhaitable que l'agent de blocage soit éliminé du film de composition cosmétique déposé et chauffé. Cette élimination peut se faire par exemple par évaporation de l'agent de blocage.
Dans un mode de réalisation préféré de l'invention, l'agent de blocage BH a par conséquent un point d'ébullition supérieur à 45 °C et inférieur ou égal à 100 °C, et en particulier compris entre 45 °C et 80 °C.

Les composés (b) portant au moins deux fonctions isocyanate bloquées sont obtenus par réaction entre un agent de blocage BH approprié et un composé comportant au moins deux fonctions isocyanate libres. Ces composés comportant au moins deux fonctions isocyanate libres sont connus dans la technique. Il peut s'agir de diisocyanates ou de polyisocyanates de faible masse moléculaire, ou bien d'oligomères ou de polymères synthétiques de toute nature chimique, obtenus par polyaddition, polycondensation et/ou greffage, ou de polymères d'origine naturelle, éventuellement modifiés chimiquement, portant deux ou plus de deux fonctions isocyanate soit aux extrémités de chaîne soit sur les groupes latéraux.

On peut citer à titre d'exemples non limitatifs de tels composés :
a) les diisocyanates aliphatiques, cycloaliphatiques et/ou aromatiques ayant notamment de 4 à 100, de préférence de 4 à 30 atomes de carbone, tels que l'hexaméthylènediisocyanate, l'isophoronediisocyanate, le toluène-diisocyanate et le diphénylméthanediisocyanate,
b) les triisocyanates aliphatiques, cycloaliphatiques et/ou aromatiques ayant notamment de 4 à 50, de préférence de 4 à 30 atomes de carbone, tels que
   - les triisocyanates obtenus par réaction entre un triol et un excès de diisocyanate, notamment ceux de formule : 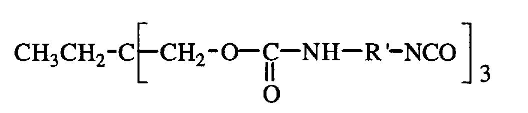
   - les isocyanatobiurets de formule : 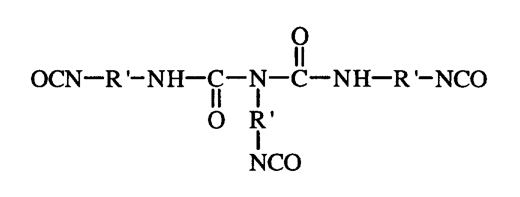
   - les isocyanurates de formule : 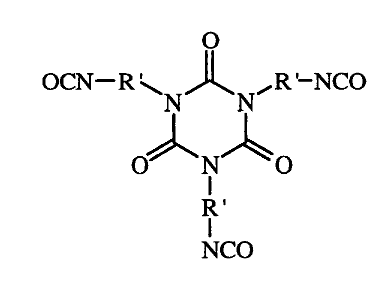
   où chaque R' représente indépendamment un radical hydrocarboné linéaire, ramifié ou cyclique comportant de 2 à 30 atomes de carbone.
   De tels triisocyanates non bloqués sont commercialisés par exemple sous la dénomination DESMODUR® L et DESMODUR® N par la société BAYER et sous la dénomination TOLONATE® HDB-LV par la société RHODIA.
c) les polycondensats à groupes isocyanate terminaux ou latéraux, tels que les polyuréthannes, les polyurées, les polyéthers, les polyesters, les polyamides et les perfluoropolyéthers. De tels polycondensats à groupes isocyanate sont notamment décrits dans les documents US-A-5 281 654, US-A-6 106 578, US-A-6 100 310, WO 99/48942, CN-A-1 093 377, JP-A-04-077581 et FR-A-1 573 596.
d) les polymères résultant de la copolymérisation de monomères vinyliques, allyliques et/ou (méth)acryliques et de comonomères à insaturation éthylénique comportant une fonction isocyanate libre, tels que le méthacrylate d'isocyanatoéthyle.
e) les silicones à groupes isocyanate notamment les silicones aminées à groupes isocyanate telles que celles décrites dans EP-A-814764.

Des composés à groupements isocyanate bloqués sont commercialisés sous des dénominations VESTANAT® B1358A, VESTANAT® B1370, VESTANAT® B1358/100 par la société CREANOVA, sous les dénominations TOLONATE® D2 ou D2R565 par la société RHODIA, sous la dénomination DESMODUR® Z4470 par la société BAYER et sous les dénominations TRIXENE® B 1 7951 et TRIXENE® B 1 7982 par la société BAXENDEN.

On peut également utiliser, comme composés (b), des composés à fonctions isocyanates "autobloquées", tels que les uréthanne-diones obtenues par dimérisation de 2 molécules de diisocyanates, ou encore les tris((alcoxy en C₁₋₆)-carbonylamino)triazines telles que le produit de condensation de mélanine, de carbonate de diméthyle et de butanol. De tels composés sont notamment vendus sous le nom de CYLINK® 2000 par la société CYTECK.

Les composés (a) portant au moins deux fonctions à hydrogène labile utilisés dans la présente invention sont également connus. Il peut s'agir de composés organiques de faible masse moléculaire ou bien d'oligomères ou de polymères synthétiques, obtenus par polyaddition, polycondensation et/ou greffage, ou de polymères naturels modifiés chimiquement.
On peut citer comme familles de composés, les diols et polyols, les diamines et polyamines primaires et/ou secondaires, les aminoalcools et les polymères comportant au moins deux fonctions à hydrogène labile.
Des exemples particuliers de composés (a) sont : les alkylèneglycols en C₁₋₄, le glycérol, le triméthylolpropane, le pentaérythritol, les poly(alkylène en C₁₋₄)glycols tels que le polyéthylèneglycol ou polyproylèneglycol ou des copolymères de ceux-ci, le produit de condensation de propylèneglycol et de triméthylolpropane, l'huile de ricin, le phytantriol, les sucres et carbohydrates tels que le saccharose ou la cellulose, l'éthylènediamine, le 1,3-diaminopropane, la lysine, l'amino-2-méthyl-2-propanol-1, les poly(alkylèneoxy)diamines telles que les produits JEFFAMINE® commercialisés par la société TEXACO, la nitrocellulose, les esters de cellulose, notamment ceux ayant un degré de substitution inférieur à 3, tels que l'acétobutyrate de cellulose et l'acétopropionate de cellulose, les éthers de cellulose tels que l'hydroxyéthylcellulose, la carboxyméthylcellulose, l'hydroxypropylcellulose ou l'éthylcellulose, les résines polyesters, silicones, perfluoropolyéthers, alkydes et polycétones à extrémités hydroxylées, le poly(alcool vinylique) et les copolymères à base d'alcool vinylique, les copolymères d'alcool allylique, les copolymères à base de (méth)acrylate d'hydroxyalkyle en C₂₋₁₀, comme le (méth)acrylate de 2-hydroxyéthyle ou de 2-hydroxypropyle, vendus notamment sous la dénomination JONCRYL® SCX 910 par la société JOHNSON POLYMER ou sous la dénomination CRODOPLAST® AC 5725 par la société CRODA, les copolymères à base de vinylamine ou d'allylamine, les silicones et les perfluoroéthers à extrémités amine primaire ou secondaire, les dendrimères ou polymères hyperramifiés à extrémités hydroxyle ou amine primaire tels que les polyesters hyperramifiés à extrémités hydroxyle commercialisés par la société PERSTORP sous les dénominations BOLTORN® H40 TMP CORE et HBP POLYOL® 3G (décrits dans les demandes internationales WO 93/17060 et WO 96/12754), ou encore les dendrimères de type polyamido-amines à extrémités amine primaire décrits dans l'article de Tomalia, Angewandte Chemie, Int. Engl. Ed., vol. 29, n° 2, pages 138 - 175.

La concentration des composés (a) et (b) dans les compositions cosmétiques de la présente invention est de préférence comprise entre 1 % et 50 % en poids, de préférence entre 2 % et 40 % en poids.
Par ailleurs, les composés (a) et les composés (b) sont présents en des quantités telles que le rapport du nombre de fonctions à hydrogène labile au nombre de fonctions isocyanate bloquées soit supérieur ou égal à 1, notamment compris entre 1 et 1,5

Le système chimique de réticulation contenant les composés (a) et (b) décrit ci-dessus est stable à température ambiante et peut être activé par une augmentation de la température. La température nécessaire pour activer les fonctions isocyanates bloquées dépend bien entendu de la nature chimique de l'agent bloquant et de celle du di- ou polyisocyanate. Lorsque la température minimale d'activation d'un certain type de fonction bloquée s'avère trop importante, c'est-à-dire incompatible avec des substrats biologiques, il est possible d'abaisser cette température d'activation par addition de catalyseurs appropriés.
La gamme des températures utilisées pour le durcissement des films va de préférence de 45 ° à 150 °C, et en particulier de 50 °C à 100 °C.

Les compositions cosmétiques thermoréticulables de la présente invention contiennent donc de préférence un ou plusieurs catalyseurs capables d'accélérer la réaction de déblocage thermique des fonctions isocyanate bloquées. Ces catalyseurs sont choisis notamment parmi les amines tertiaires telles que le diazabicyclo[2,2,2]octane, la quinuclidine et le 3,3,6,9,9-pentaméthyl-2,10-diazabicyclo[4,4,0]déc-1-ène, le chlorure d'étain, les composés organométalliques tels que les acétonylacétates métallliques, les composés organométalliques de l'étain, l'hexanoate de calcium, le 2-éthylhexanoate de calcium, l'octanoate de calcium et le linoléate de calcium, le dibutyldilaurate d'étain, le tris(2-éthylhexanoate) de bismuth et le bis(2-éthylhexanoate) de zinc.
Ces catalyseurs et les conditions de leur utilisation sont également décrits dans l'article "Blocked Isocyanates" de Zeno W. dans *Progress in Organic Coatings*, 3, pages 73- 99 (1975)
Selon la présente invention, la concentration des catalyseurs est de préférence comprise entre 0,1 et 5 % en poids, et plus particulièrement entre 0,2 et 3 % en poids, rapportée au poids total du composé (b) porteur des groupes isocyanates bloqués.

Les compositions cosmétiques thermoréticulables de la présente invention peuvent contenir un ou plusieurs solvants, de préférence volatils, choisis parmi l'eau et les solvants organiques physiologiquement acceptables parmi lesquels on peut citer
- les cétones liquides à température ambiante telles que la méthyléthylcétone, la méthylisobutylcétone, la diisobutylcétone, l'isophorone, la cyclohexanone et l'acétone,
- les alcools liquides à température ambiante tels que l'éthanol, l'isopropanol, le diacétone-alcool, le 2-butoxyéthanol ou le cyclohexanol,
- les glycols liquides à température ambiante tels que l'éthylèneglycol, le propylèneglycol, le pentylèneglycol et le glycérol,
- les éthers de propylèneglycol liquides à température ambiante tels que le monométhyléther de propylèneglycol, l'acétate de l'éther monométhylique de propylèneglycol, le mono-n-butyléther de dipropylèneglycol,
- les esters à courte chaîne (comportant au total de 3 à 8 atomes de carbone) tels que l'acétate d'éthyle, l'acétate de méthyle, l'acétate de propyle, l'acétate de n-butyle et l'acétate d'isopentyle,
- les alcanes liquides à température ambiante tels que le décane, l'heptane, le dodécane, le cyclohexane,
- les hydrocarbures aromatiques liquides à température ambiante tels que le toluène et le xylène,
- les silicones liquides à température ambiante et
- des mélanges de ceux-ci.

La teneur en solvant dans la composition peut aller de 0,1 % à 80 % en poids, par rapport au poids total de la composition, et de préférence de 1 à 60 % en poids.
Selon un mode particulier de la composition selon l'invention, la composition est exempte de solvant.

Les compositions cosmétiques photoréticulables de la présente invention peuvent contenir en outre des adjuvants et additifs utilisés couramment dans des vernis à ongles, choisis notamment parmi les pigments et colorants, les agents plastifiants, les agents de coalescence, les agents conservateurs, les cires, les agents épaississants, les parfums, les filtres UV, les actifs cosmétiques pour le soin des ongles, les agents d'étalement, les agents anti-mousses, les agents tensioactifs et les agents dispersants.

Bien entendu l'homme du métier veillera à choisir ces éventuels adjuvants et additifs de manière à ce que les propriétés avantageuses des compositions selon l'invention ne soient pas, ou pratiquement pas altérées par l'adjonction envisagée.

L'invention a également pour objet un procédé de revêtement de substrats kératiniques. Ce procédé comprend généralement les étapes consistant
- à appliquer sur le substrat kératinique une couche d'une composition cosmétique thermoréticulable décrite ci-dessus,
- à laisser éventuellement sécher la composition cosmétique déposée, et
- à soumettre la composition cosmétique déposée, éventuellement séchée, à un chauffage jusqu'à une température suffisante et pendant une durée suffisante pour provoquer le déblocage d'une partie ou de la totalité des fonctions isocyanate bloquées portées par le composé (a), de manière à permettre la réticulation partielle ou totale du dépôt.

Les substrats kératiniques susceptibles de recevoir des revêtements thermoréticulables selon la présente invention sont en particulier les ongles, les cheveux, les cils et les sourcils, mais on peut également revêtir les accessoires de maquillage tels que les faux-cils, les faux-ongles ou les pastiches.

Le taux de réticulation des revêtements cosmétiques selon la présente invention ne dépend pas seulement de la température, mais bien entendu également de la durée du chauffage. Plus celle-ci est longue, moins la température minimale nécessaire à la réticulation est faible.
La température de chauffage, c'est-à-dire la température locale obtenue *in situ* dans la couche de composition cosmétique déposée, est de préférence comprise entre 45 °C et 150 °C, et en particulier entre 50°C et 100 °C, cette température étant maintenue de préférence pendant environ 2 minutes à 1 heure, et en particulier pendant 5 et 15 minutes.

Le chauffage de la couche de composition cosmétique peut se faire au moyen de toute source de chaleur appropriée. On peut citer à titre d'exemple d'une telle source de chaleur une enceinte chauffante, un appareil propulsant de la chaleur tel qu'un sèche-cheveux, ou une source de rayonnement permettant d'élever la température de la composition telle qu'une lampe infrarouge.

L'utilisation d'une lampe infrarouge représente un mode de réalisation préféré de l'invention car elle permet d'obtenir des températures locales élevées au niveau de la couche de verni déposée sans que cela n'implique un chauffage excessif du support cosmétique, c'est-à-dire de l'ongle ou des cheveux. L'utilisation d'une lampe infrarouge permet également d'obtenir une réticulation différentielle de la couche déposée, c'est-à-dire un dépôt plus réticulé en surface qu'en profondeur, ce qui donne des revêtements présentant une bonne résistance mécanique, une bonne adhésion au support et qui s'éliminent facilement par des dissolvants classiques.

L'invention est décrite plus en détail grâce aux exemples ci-dessous.

### Exemples

### Exemple 1

On prépare une composition de vernis à ongles à partir des ingrédients suivants :

| | |
|---|---|
| JONCRYL® SCX 910 (Johnson Polymer) (polymère acrylique à groupes hydroxyle) | 21 g |
| TOLONATE® D2 (Rhodia) (polyisocyanate bloqué par méthyléthylcétone) | 9 g |
| AEROSIL® R972 (Degussa) (silice pyrogénée) | 0,5 g |
| BYK® 162 (Byk) (agent tensioactif) | 1 g |
| MODAFLOW® (Monsanto) (agent tensioactif) | 0,1 g |
| Pigments | 3 g |
| Acétate de butyle | 20 g |
| Acétate d'éthyle | qsp 100 g |

On applique un film de cette composition sur l'ongle, on le laisse sécher pendant environ 5 minutes de manière à laisser le solvant s'évaporer. On élève ensuite la température du film à 65 °C pendant 3 minutes à l'aide d'un sèche-cheveux.
Le film obtenu est sec au toucher, non collant, brillant et il présente de bonnes propriétés d'adhésion au substrat et une bonne tenue dans le temps.

### Exemple 2

On prépare une composition de vernis à ongles à partir des ingrédients suivants :

| | |
|---|---|
| CRODOPLAST® AC 5725 (Croda) (polymère acrylique à groupes hydroxyle) | 16,4 g |
| TRIXÈNE® B 1 7951 (Baxenden) (isophoronediisocyanate bloqué par 3,5-diméthylpyrazole) | 7,1 g |
| TRIXÈNE® B 1 7982 (Baxenden) (hexaméthylènediisocyanate bloqué par 3,5-diméthylpyrazole) | 6,5 g |
| Dibutyldilaurate d'étain | 0,01 g |
| AÉROSIL® R972 (Dégussa) (silice pyrogénée) | 0,5 g |
| Pigments | 3 g |
| MODAFLOW® (Monsanto) (agent tensioactif) | 0,1 g |
| Heptane | 25 g |
| Acétate d'éthyle | qsp 100 g |

On applique un film de cette composition sur les ongles, on le laisse sécher pendant environ 5 minutes de manière à laisser le solvant s'évaporer. On élève alors la température de la surface du film au moyen d'un rayonnement infrarouge qui donne des impulsions régulières d'une température d'au moins 80 °C pendant quelques secondes pour initier le déblocage des fonctions isocyanate. Le film obtenu présente des propriétés identiques à celles du film obtenu dans l'exemple 1.

## Revendications

1. Composition cosmétique thermoréticulable comprenant, dans un milieu cosmétiquement acceptable,
(a) au moins un composé comportant au moins deux fonctions à hydrogène labile, et
(b) au moins un composé comportant au moins deux fonctions isocyanate bloquées, susceptibles d'être débloquées par chauffage,
la fonctionnalité moyenne du système, c'est-à-dire le nombre total de fonctions à hydrogène labile et de fonctions isocyanate bloquées rapporté au nombre total de molécules de composés (a) et (b), étant strictement supérieure à 2.

2. Composition cosmétique thermoréticulable selon la revendication 1, **caractérisée par le fait qu'**une partie ou la totalité des composés (a) portent également une ou plusieurs fonctions isocyanate bloquées et/ou qu'une partie ou la totalité des composés (b) portent également une ou plusieurs fonctions à hydrogène labile.

3. Composition cosmétique thermoréticulable selon la revendication 1 ou 2, **caractérisée par le fait que** les fonctions à hydrogène labile, portées par le composé (a), sont choisies parmi les fonctions amine primaire (-NH₂), amine secondaire (>NH), hydroxyle (-OH), acide carboxylique (-COOH) ou thiol (-SH).

4. Composition cosmétique thermoréticulable selon les revendications 1 à 3, **caractérisé par le fait que** les fonctions isocyanate bloquées du composé (b) répondent à la formule
-NH-C(=O)-B
où B représente un radical dérivé d'un agent de blocage BH choisi parmi les composés organiques comportant un ou plusieurs, et de préférence un seul atome d'hydrogène labile.

5. Composition cosmétique thermoréticulable selon la revendication 4, **caractérisée par le fait que** l'agent de blocage BH est choisi parmi les monoalcools, les monophénols, les amides, les oximes, les composés β-dicarbonyle, les pyrazoles, les esters d'acide hydroxyamique et d'alcools en C₁₋₆, les triazoles, les imidazolines, les tétrahydropyrimidines et les imidazoles.

6. Composition cosmétique thermoréticulable selon la revendication 4 ou 5, **caractérisée par le fait que** l'agent de blocage BH a un point d'ébullition supérieur à 45 °C et inférieur ou égal à 100 °C, de préférence comprise entre 45 °C et 80 °C.

7. Composition cosmétique thermoréticulable selon l'une quelconque des revendications précédentes, **caractérisée par le fait que** le composé (b) portant au moins deux fonctions isocyanate bloquées est obtenu par réaction entre un agent de blocage BH définit dans les revendications 4 à 6 et un composé comportant au moins deux fonctions isocyanate choisi parmi
a) les diisocyanates aliphatiques, cycloaliphatiques et/ou aromatiques ayant notamment de 4 à 50, de préférence de 4 à 30 atomes de carbone, tels que l'hexaméthylènediisocyanate, l'isophoronediisocyanate, le toluène-diisocyanate, le diphénylméthanediisocyanate,
b) les triisocyanates aliphatiques, cycloaliphatiques et/ou aromatiques ayant notamment de 4 à 100, de préférence de 4 à 30 atomes de carbone, tels que ceux de formule 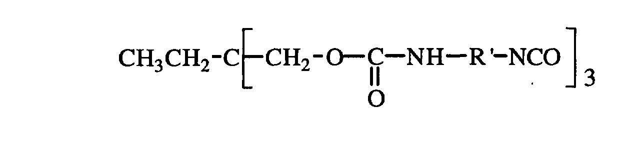 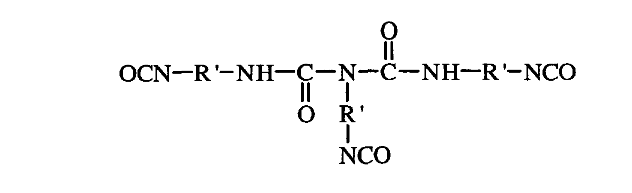 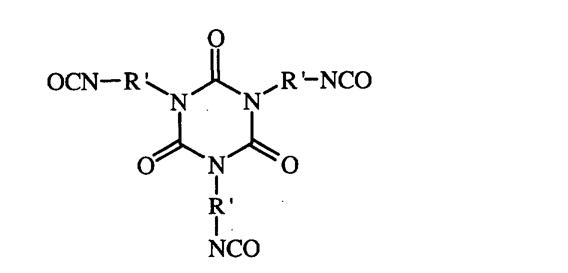 où chaque R' représente indépendamment un radical hydrocarboné linéaire, ramifié ou cyclique comportant de 2 à 30 atomes de carbone.
c) les polycondensats à groupes isocyanate terminaux ou latéraux, tels que les polyuréthannes, les polyurées, les polyéthers, les polyesters, les polyamides et les perfluoropolyéthers.
d) les polymères résultant de la copolymérisation de monomères vinyliques, allyliques et/ou (méth)acryliques et de comonomères à insaturation éthylénique comportant une fonction isocyanate libre,
e) les silicones à groupes isocyanate.

8. Composition cosmétique thermoréticulable selon l'une quelconque des revendications précédentes, **caractérisée par le fait que** le composé (a) est choisi parmi les diols et polyols, les diamines et polyamines primaires et/ou secondaires, les aminoalcools et les polymères comportant au moins deux fonctions à hydrogène labile.

9. Composition cosmétique thermoréticulable selon la revendication 8, **caractérisée par le fait que** le composé (a) est choisi parmi les alkylèneglycols en C₁₋₄, le glycérol, le triméthylolpropane, le pentaérythritol, les poly(alkylène en C₁₋₄)glycols tels que le polyéthylèneglycol ou polyproylèneglycol ou des copolymères de ceux-ci, le produit de condensation de propylèneglycol et de triméthylolpropane, l'huile de ricin, le phytantriol, les sucres et carbohydrates tels que le saccharose ou la cellulose, l'éthylènediamine, le 1,3-diaminopropane, la lysine, l'amino-2-méthyl-2-propanol-1, les poly(alkylèneoxy)diamines, la nitrocellulose, les esters de cellulose, les éthers de cellulose, les résines polyesters, silicones, perfluoropolyéthers, alkydes et polycétones à extrémités hydroxylées, le poly(alcool vinylique) et les copolymères à base d'alcool vinylique, les copolymères d'alcool allylique, les copolymères à base de (méth)acrylate d'hydroxyalkyle en C₂₋₁₀, les copolymères à base de vinylamine ou d'allylamine, les silicones et les perfluoroéthers à extrémités amine primaire ou secondaire, les dendrimères ou polymères hyperramifiés à extrémités hydroxyle ou amine primaire.

10. Composition cosmétique thermoréticulable selon l'une quelconque des revendications précédentes, **caractérisée par le fait que** les composés (a) et (b) représentent de 1 à 50 % en poids de la composition cosmétique.

11. Composition cosmétique thermoréticulable selon l'une des revendications précédentes, **caractérisée par le fait que** le nombre total de fonctions à hydrogène libre et de fonctions isocyanate bloquées rapporté au nombre total de molécules de composés (a) et (b) est supérieur à 2,2, de préférence comprise entre 2,5 et 100.

12. Composition cosmétique thermoréticulable selon l'une quelconque des revendications précédentes, **caractérisée par le fait qu'**elle comprend en outre un ou plusieurs composés permettant de catalyser la réaction de déblocage thermique des fonctions isocyanate du composé (b), choisis parmi les amines tertiaires telles que le diazabicyclo[2,2,2]octane, la quinuclidine et le 3,3,6,9,9-pentaméthyl-2,10-diazabicyclo[4,4,0]déc-1-ène, le chlorure d'étain, les composés organométalliques tels que les acétonylacétates métallliques, les composés organométalliques de l'étain, l'hexanoate de calcium, le 2-éthylhexanoate de calcium, l'octanoate de calcium et le linoléate de calcium, le dibutyldilaurate d'étain, le tris(2-éthylhexanoate) de bismuth et le bis(2-éthylhexanoate) de zinc.

13. Composition cosmétique thermoréticulable selon la revendication 12, **caractérisée par le fait que** la concentration du composé permettant de catalyser la réaction de déblocage thermique des fonctions isocyanate du composé (b) est comprise entre 0,1 et 5 % en poids, de préférence entre 0,2 et 3 % en poids, rapportée au poids total de composé (b) présent.

14. Procédé de revêtement d'un substrat kératinique, comprenant les étapes consistant
- à appliquer sur le substrat kératinique une couche d'une composition cosmétique selon une des revendications précédentes,
- à laisser éventuellement sécher la composition cosmétique déposée, et
- à soumettre la composition cosmétique déposée, éventuellement séchée, à un chauffage jusqu'à une température suffisante et pendant une durée suffisante pour provoquer le déblocage d'une partie ou de la totalité des fonctions isocyanate bloquées portées par le composé (a), de manière à permettre la réticulation du dépôt.

15. Procédé selon la revendication 14, **caractérisé par le fait que** le substrat kératinique est l'ongle, les cheveux, les cils et les sourcils.

16. Procédé selon l'une des revendications 14 et 15, **caractérisé par le fait que** la composition cosmétique est chauffée jusqu'à une température comprise entre 45 °C et 150 °C, de préférence comprise entre 50 et 100 °C.

17. Procédé selon l'une des revendications 14 à 16, **caractérisé par le fait que** la durée de chauffage est comprise entre 2 minutes et 1 heure, de préférence entre 5 et 15 minutes.

18. Procédé selon l'une des revendications 14 à 17, **caractérisé par le fait que** le chauffage se fait au moyen d'une source de chaleur choisie parmi une enceinte chauffante, un appareil propulsant de la chaleur tel qu'un sèche cheveux, ou une source de rayonnement permettant d'élever la température de la composition telle qu'une lampe infrarouge.
